# EUROPEAN PATENT SPECIFICATION

(11) **EP 1 893 630 B1**
(45) Date of publication and mention of the grant of the patent: **02.09.2009**
(21) Application number: 06763763.7
(22) Date of filing: 16.06.2006
(51) Int. Cl.: C07J 63/00, A61K 31/565, A61P 5/26

(54) **STEROIDS HAVING A MIXED ANDROGENIC AND PROGESTAGENIC PROFILE**
STEROIDE MIT EINEM ANDROGENEN UND PROGESTAGENEN MISCHPROFIL
STÉROÏDES AYANT UN PROFIL MIXTE ANDROGÈNE ET PROGESTAGÈNE

(30) Priority: 17.06.2005 EP 05076437
(43) Date of publication of application: 05.03.2008
(73) Proprietor: N.V. Organon, 5349 AB Oss (NL)
(72) Inventor: VAN DER LOUW, Jaap, N.V. Organon, NL-5340 BH Oss (NL); LEYSEN, Dirk, B-9160 Lokeren (BE); DE GOOIJER, Marcel Evert, N.V. Organon, NL-5340 BH Oss (NL); CALS, Joseph Maria Gerardus Barbara, N.V Organon, NL-5340 BH Oss (NL)
(74) Representative: Broekkamp, Chris L. E.
(86) International application number: PCT/EP2006/063282
(87) International publication number: WO 2006/134167

(56) References cited:
- WO-A-85/05361
- US-A- 3 577 410
- US-A- 3 959 322
- US-A1- 2004 059 140
- AVERY M A ET AL: "Synthesis and testing of 17a.beta.-hydroxy-7.alpha.-methyl- D-homoestra-4,16-dien-3-one: a highly potent orally active androgen" STEROIDS: STRUCTURE, FUNCTION, AND REGULATION, ELSEVIER SCIENCE PUBLISHERS, NEW YORK, NY, US, vol. 55, no. 2, February 1990 (1990-02), pages 59-64, XP002118688 ISSN: 0039-128X

## Description

The invention relates to compounds having a mixed profile of androgenic and progestagenic effects, to pharmaceutical compositions comprising these compounds and to their use in therapy.

Male contraception seeks to suppress spermatogenesis through the suppression of the gonadotropins luteinizing hormone (LH) and follicle-stimulating hormone (FSH). This results in a depletion of intratesticular testosterone and cessation of spermatogenesis.

Administration of progestagen results in a dose dependent suppression of pituitary gonadotrophins and consequently, a decrease in testosterone levels and a reversible inhibition of spermatogenesis. An exogenous androgen is required to compensate for the reduced testosterone levels.

Analogously, male hormone replacement therapy (HRT) can be accomplished, resulting in replacement of testosterone by an exogenous androgen which is safer on the prostate than endogenous testosterone.

Both for male contraception and male HRT it would be particularly useful to employ a compound having a mixed profile of androgenic and progestagenic activities, *i*.*e*. a compound which possesses both activities intrinsically within one molecule. The aim would be to provide compounds with optimal potency, oral activity, and safety. With regard to safety it is particularly important to minimize the risk of bone loss or liver toxicity.

Compounds with both androgenic and progestagenic properties are known in the art. For example, 7α,17α-dimethylnandrolone (Mibolerone) is a highly active androgen also having progestagenic activity (see e.g. L. Markiewicz et al., Methods and Findings in Experimental and Clinical Pharmacology (1997), 19(4), 215-222). It is orally active but suffers from the drawback of severe liver toxicity (see *e.g.*, J. Seaman, Toxicologic Pathology (1985), 13(3), 177-180).

Another example is (7α,17β)-17-hydroxy-7,17-dimethylestra-4,14-dien-3-one (US 3,577,410) which is reported to have androgenic activity. It suffers, however, from severe liver toxicity.

Certain D-homosteroids are also known to possess a mixed androgenic and progestagenic profile. For instance, (17aβ)-13-ethyl-17a-hydroxy-D-homogon-4-en-3-one, and (17aβ)-13-ethyl-17a-hydroxy-17a-methyl-D-homogon-4-en-3-one, which are described in US 3,959,322, are reported to possess both androgenic and progestagenic activity. We have found, however, that these compounds are not orally active.

7α-Methyl-19-nor-D-homotestosterone is disclosed as a synthetic intermediate in WO 85/05361. We found that it is not an orally active compound.

17aα-Methyl-19-nor-D-homotestosterone, indicated to possess anabolic activity, is described by S.N. Ananchenko et al. in Tetrahedron (1962), 18, 1355-67. We have found, however, that the compound suffers from a combination of low activity upon oral administration and is unable to prevent trabecular bone mineral density (BMD) loss.

Δ¹⁵-D-Homosteroids are named in WO 2005/021573 in the context of androgenic compounds.

US 2004/0059140 discloses 14(15) -unsaturated androgen- related steroids which present a mixed profile of androgen and progestagenic activities.

It has now been found, unexpectedly, that within a series of 19-nor-D-homosteroids, steroids with mixed, androgenic/progestagenic effect exist, which, by comparison to the compounds of the prior art have also improvements with respect to oral activity or reduced liver toxicity. Another advantage can reside in improved suitability for transdermal administration. Thus, the invention provides compounds according to formula I: 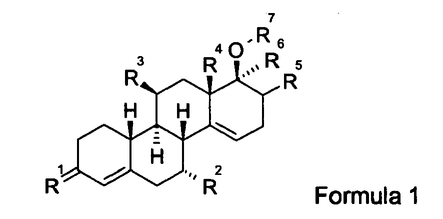 wherein,
R¹ is O, (H,H), (H,OH), or NOR, with R being hydrogen, (C₁₋₆) alkyl or (C₁₋₆) acyl;
R² is hydrogen, methyl, ethyl, or ethenyl;
R³ is hydrogen, methyl, ethyl, ethenyl, fluoro, or chloro;
R⁴ is methyl, or ethyl;
R⁵ is hydrogen, methyl, or ethyl;
R⁶ is hydrogen, methyl, ethyl, (C₂₋₃) alkenyl, or (C₂₋₃) alkynyl;
R⁷ is hydrogen or (C₁₋₁₅) acyl;
and at least one of R⁵ or R⁶ is hydrogen;
and pharmaceutically acceptable salts thereof.

A specific embodiment of the invention is provided by compounds in which R¹ is O, or (H,OH), R² is hydrogen, methyl, ethyl, R³ is hydrogen, methyl, R⁴ is methyl, R⁵ is hydrogen, methyl, R⁶ is hydrogen, methyl, ethyl, or (C₂₋₃) alkynyl and R⁷ is hydrogen or (C₁₋₁₅) acyl.
Another more specific embodiment of the invention is provided by compounds in which R¹ is O, or (H,OH), R² is hydrogen, methyl, R³ is hydrogen, R⁴ is methyl, R⁵ is hydrogen, R⁶ is hydrogen, methyl, ethyl, or ethynyl and R⁷ is hydrogen or (C₁₋₁₅) acyl.
Other more specific embodiments of the invention are provided by compounds according to the embodiments defined above, wherein, in addition, R¹ is O.

The compounds wherein R¹ is (H,H) or NOR, with R being hydrogen, (C₁₋₆) alkyl or (C₁₋₆) acyl, are prodrugs of the compounds wherein R¹ is O. Likewise, the compounds wherein R⁷ is (C₁₋₁₅) acyl are prodrugs of the corresponding compounds wherein R⁷ is hydrogen. Said prodrugs are derivatives, which are converted in vivo to other compounds according to the invention.

The Δ¹⁴-D-homo derivatives of this invention have the natural configurations 8β, 9α, 10β, and 13β. The configuration at C-7 is 7α and the configuration at C-17a is 17aβ with respect to OR⁷.

The term (C₁₋₆) alkyl represents a branched or unbranched alkyl group having 1-6 carbon atoms. Examples of (C₁₋₆) alkyl groups include methyl, ethyl, propyl, isopropyl, butyl, and tertiary butyl.

The term (C₂₋₃) alkenyl represents a branched or unbranched alkenyl group having 2-3 carbon atoms. Examples of (C₂₋₃) alkenyl groups include ethenyl, 1-propenyl, and 2-propenyl.

The term (C₂₋₃) alkynyl represents an alkynyl group having 2-3 carbon atoms. Examples of (C₂₋₃) alkynyl groups include ethynyl and 1-propynyl.

The term (C₁₋₆) acyl and (C₁₋₁₅) acyl represent an acyl group derived from a carboxyl ic acid having respectively 1-6 and 1-15 carbon atoms. The acyl group can comprise a hydrocarbon which may be branched, unbranched, saturated or unsaturated. Examples of (C₁₋₆) acyl groups include formyl, acetyl, propanoyl, propenoyl and pivaloyl, and examples of (C₁₋₁₅) acyl include decanoyl and undecanoyl. Also included within the definition of (C₁₋₆) acyl and (C₁₋₁₅) acyl are acyl groups derived from dicarboxylic acids like hemi-maloyl, hemi-succinoyl and hemi-glutaroyl.

The prefixes (C₁₋₁₅), (C₂₋₃) etc. have the usual meaning to restrict the meaning of the indicated group to those with 1 to 15, 2 to 3 etc. carbon atoms;

In those cases that a compound of the invention contains a free carboxylic acid function, the compound may be used as a free acid or as a pharmaceutically acceptable salt such as a sodium or potassium salt.

Biological activity of compounds according to the invention can be observed in vivo in the rat serum LH suppression test and in the rat anti-osteoporosis test measuring restoration of trabecular bone mineral density. Anabolic effect can be observed on musculus levator ani. In the monkey activity can be observed in the testosterone suppression test. In vivo liver safety can be evaluated in the rabbit upon repeated administration of a compound according to the invention.

The biological activity and safety of the compounds according to the invention can be exploited in animals and humans for therapy, i.e. for the treatment and/or prevention of disease states as well as in treatment regimes to achieve male contraception. Diseases in which a compound according to the invention can be used are those in which endogenous androgen levels are too low (which is androgen insufficiency) or are in need to be increased by treatment with androgens. Compounds according to the invention can also be used in diagnostic methods focussed on the amount and/or location of androgen receptors in various tissues. Treatments to remedy low levels of endogenous androgen are, for example androgen supplementation as well as testosterone replacement, often also indicated as hormone replacement therapy (HRT). Such a treatment is useful for the partially androgen deficient (ageing) male. Furthermore, the compounds of the invention can be used for the treatment of benign prostate hypertrophy (BPH), osteoporosis, as well as other bone disorders, bone fraction repair, sarcopenia, frailty, skin aging, female sexual dysfunction, postmenopausal symptoms, atherosclerosis, aplastic anemia, muscular atrophy, lipodystrophy, reduced muscle strength and function, side effects of chemotherapy, chronic fatigue syndrome, cachexia, chronic catabolic state or cognitive impairment. For the diagnostic purpose it can be preferred to make labelled variants of the compounds according to the invention. Androgen receptor-related treatments can also be for female hormone replacement therapy. In the context of the invention, the term "androgen insufficiency" is to be understood to pertain to all kinds of diseases, disorders, and symptoms in which a male or a female suffers from too low a testosterone level, such as in hypogonadal men or boys. In particular, the androgen insufficiency to be treated by a compound of the invention is the reduction of the testosterone level which a male individual incurs as a result of age (the compound of the invention is then used for male hormone replacement therapy), or when he is subject to male contraception. In the context of male contraception, the compound of the invention especially serves to neutralise the effect of regimens of male hormone contraception in which a sterilitant such as a progestagen or LHRH (luteinizing hormone releasing hormone) is administered regularly, *e.g.* daily, or it is used as the sole male contraceptive substance.

Male contraception frequently is described to comprise a regimen of administration of hormones in which a progestagen serves to achieve a contraceptive effect and an androgen serves to supplement the resulting decreased testosterone level. The combined androgenic/progestagenic nature of the compounds of the invention allows the achievement of male contraception through the progestagen-androgen system on the basis of one single compound.

When the androgenic/progestagenic compounds of the invention are used for (partial) replacement of endogenous testosterone, the progestagenic activity leads to an advantage of the compounds of the invention in that the production of endogenous testosterone is suppressed. The androgenic activity serves to compensate for the resulting testosterone deficiency. This allows replacement of testosterone by an exogenous androgen, which is safer than natural testosterone. Natural testosterone is converted by 5α-reductase to the more potent 5α-dihydrotestosterone resulting in well known detrimental effects such as prostate problems, acne and hair loss. Hence by virtue of the androgenic/progestagenic compounds of the invention, one can advantageously diminish the intrinsic detrimental effects of endogenous testosterone. Therefore, and more specifically, the compounds of the invention can be used for the treatment of benign prostate hypertrophy (BPH).

Optionally, the androgenic/progestagenic compounds of the invention can be combined with an androgen known in the art which is not 5α-reduceable, such as MENT, or the androgens disclosed in WO 99/67271, WO 00/53619, WO 00/59920 or WO 01/05806, or a progestagen known in the art.

In view of the above-indicated uses the invention also relates to the use of a compound according to the invention for the manufacture of a medicament for the treatment of androgen insufficiency or a medicament for testosterone replacement. The person skilled in the art will appreciate that this use can comprise the combined administration of a compound according to the invention and an androgen or alternatively the combined use of a compound according to the invention and a progestagen. Accordingly, the invention also includes compounds to be used in a method of treatment in the field of male hormone replacement therapy comprising the administration, to a male of a compound as described hereinbefore in a suitable pharmaceutical dosage form. The invention also pertains to compounds to be used in a method of treatment comprising administering to a male in need of androgen supplementation or testosterone replacement a therapeutically effective amount of a compound according to the invention, optionally in combination with an androgen known in the art, or a progestagen known in the art.

Also, the invention relates to the use of a compound according to the invention for the manufacture of a medicament having contraceptive activity (for which in the art the term 'contraceptive agent' is also used). Said medicament can be used in a method of contraception, comprising administering to a fertile male, notably human, a compound according to the invention in a dosage amount and regimen, which is sufficient for said compound to be contraceptively effective per se, and which simultaneously serves to maintain a sufficient androgen level in the male subject to this contraceptive method. Alternatively, the method of contraception comprises administering to a fertile male, notably human, a contraceptively effective combination of a sterilitant, such as a progestagen, and a compound according to the invention. As a second alternative, the method of contraception involves the administration of a compound according to the invention as the (progestagenic) sterilitant, wherein the maintenance of a sufficient androgen level is taken care of, in part, by the androgenic activity of the compound of the invention, and which is supplemented by an additional androgen.

The invention also pertains to a kit for male contraception comprising means for the administration of a progestagen and means for the administration of an androgen, characterized in that one of the means is a pharmaceutical composition comprising a compound according to the present invention.

Administration of a compound according to the invention will be greatly aided by the manufacture of pharmaceutical compositions. The present invention therefore also relates to a pharmaceutical composition comprising a compound according to the invention mixed with a pharmaceutically acceptable excipient, such as the ones described in Gennaro et a/., Remmington: The Science and Practice of Pharmacy, 20th Edition, Lippincott, Williams and Wilkins, 2000; see especially part 5: pharmaceutical manufacturing. Suitable excipients are made available *e.g*., in the Handbook of Pharmaceutical Excipients, 2nd Edition; Editors A. Wade and P.J.Weller, American Pharmaceutical Association, Washington, The Pharmaceutical Press, London, 1994. The mixtures of a compound according to the present invention and a pharmaceutically acceptable excipient may be compressed into solid dosage units, such as tablets, or be processed into capsules or suppositories. For making dosage units *e.g*., tablets, the use of conventional additives such as fillers, colorants and polymeric binders is contemplated. In general, any pharmaceutically acceptable additive, which does not interfere with the function of the active compounds can be used. Suitable fillers with which the pharmaceutical compositions can be prepared and administered include lactose, starch, cellulose and derivatives thereof, or mixtures thereof used in suitable amounts.
Other routes of administration of the medicines comprising a compound according to the invention can be for injection into veins, subcutaneously or intra-muscularly. The compounds may also be administered with aids to obtain transdermal or transmucosal resorbtion into the body of the recipient. Transmucosal means for example by mouth, nose or rectal tissues. With sprays dosage forms for intra-oral, pulmonary of nasal administration can be prepared with methods available to the skilled person in the field of manufacture of medicines.

The compounds of the invention may be produced by various methods known in the art of organic chemistry in general, and especially in the art of the chemistry of steroids (see, for example: Fried, J. et al, Organic Reactions in Steroid Chemistry, Volumes I and II, Van Nostrand Reinhold Company, New York, 1972).
A convenient starting material for the preparation of compounds of Formula 1 is for instance a 3-methoxygona-1,3,5(10),14-tetraen-17-one derivative of general Formula 2, wherein R², R³ and R⁴ have the previously given meaning (**Scheme 1**), whose synthesis is known or can be prepared using standard methods. See for Example:
R² = H, R³ = H, R⁴ = Me (registry number 17550-11-7): WO 2002048169,
R² = Me, R³ = H, R⁴ = Me (registry number 35644-57-6): WO 2002048169,
R² = Et, R³ = H, R⁴ = Me (registry number 319003-90-2): WO 2001005806,
R² = ethenyl, R³ = H, R⁴ = Me (registry number 293303-54-5): WO 2001005806,
R² = Me, R³ = H, R⁴ = Et (registry number 343626-79-9): WO 2001040255,
R² = Et, R³ = H, R⁴ = Et (registry number 319004-25-6): WO 2001005806,
R² = ethenyl, R³ = H, R⁴ = Et (registry number 319004-43-8): WO 2001005806.
Here and elsewhere in this description of the invention Me means methyl and Et ethyl.

### Scheme 1 Experimental conditions (reagents and solvents mentioned are by way of example only): A: 1) Lithium diisopropylamide, 2) chlorotrimethylsilane. B: Diiodomethane, diethylzinc. C: Iron(III) chloride. D: Triethylsilane, boron trifluoride diethyl etherate. E: Jones reagent.

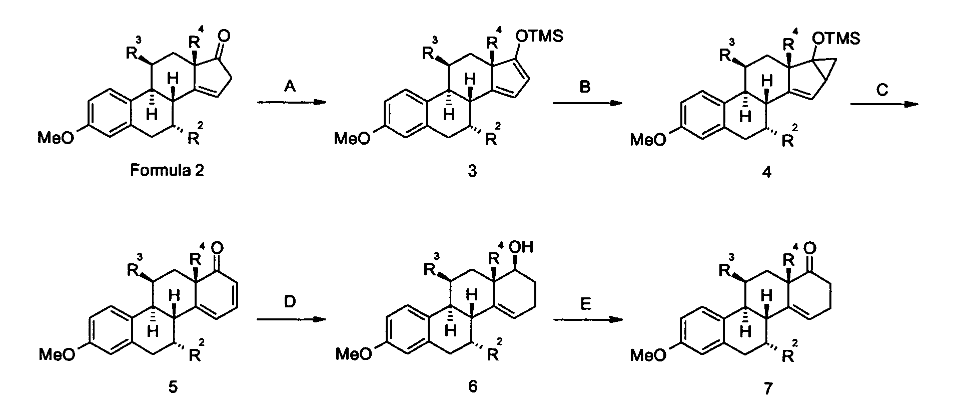

For example: a compound of Formula 2 can be converted, via enol trimethylsilyl ether **3,** to a 16, 17-methylene-17-[(trimethylsilyl)oxy]gona-1,3,5(10),14-tetraene **4 (Scheme 1).** Reaction with *e.g*. iron(III) chloride gives a D-homogona-1,3,5(10),14,16-pentaen-17a-one **5**. In the latter reaction some 16-chloro-D-homogona-1,3,5(10),14-tetraen-17a-one may also be formed which can be converted by base to **5** (not shown) [Johns, W.F. et al, J. Org. Chem. 36, 1952 (1971); Ito, Y. et al, J. Org. Chem. 41, 2073 (1976)]. Dienone **5** can be reduced in a one-pot process to alcohol **6.** The latter may be formed as a single 17aβ-OH diastereomer or together with the corresponding 17aα-OH isomer. If the 17aβ-OH diastereomer is required, they have to be separated by chromatography or crystallisation. The 17aβ-OH (or the mixture of the 17aβ-OH and the 17aα-OH isomers) can also be oxidised to ketone **7.**
Ketone **7** can also be obtained by conversion of compound of Formula 2 to 17-carbonitrile **8,** followed by reduction to amine compound **9** and reaction with sodium nitrite **(Scheme 2)** [see e.g. Avery, M.A. et al, Steroids 55, 59 (1990)].

### Scheme 2 Experimental conditions (reagents and solvents mentioned are by way of example only): A: 1) Trimethylsilyl cyanide, zinc iodide. B: Lithium aluminum hydride. C: Sodium nitrite.

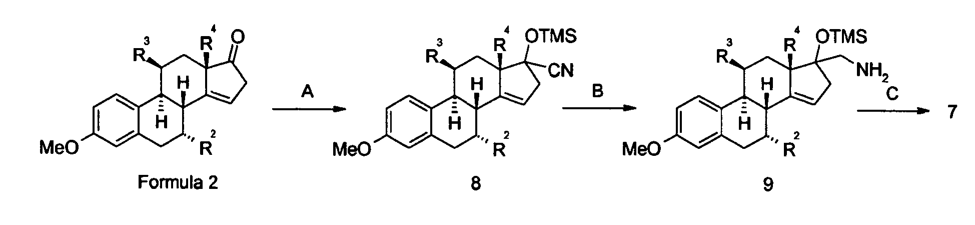

A 3-methoxy-D-homogona-1,3,5(10),14-tetraen-17a-o) **6,** prepared from dienone **5** or, optionally, *via* reduction from ketone **7 (Scheme 3),** can be reacted in a Birch reduction to give alcohol **10** [Caine, D. in Org. Reactions 23, p. 1, Wiley, New York, 1976]. The latter can also be obtained by reduction of dienone **5** and Birch reduction of the resulting dienol **11.** Hydrolysis of **10** then provides a Δ¹⁴-D-Homo compound of Formula 1 (R¹ = oxo, R⁵ = H, R⁶ = H, R⁷ = H).
Optionally, if a mixture 17a-epimers is obtained, the separation can also be carried out at this stage.

### Scheme 3 Experimental conditions (reagents and solvents mentioned are by way of example only): A: Triethylsilane, boron trifluoride diethyl etherate. B: Lithium aluminum hydride. C: Lithium/ammonia. D: Hydrochloric acid, acetone.

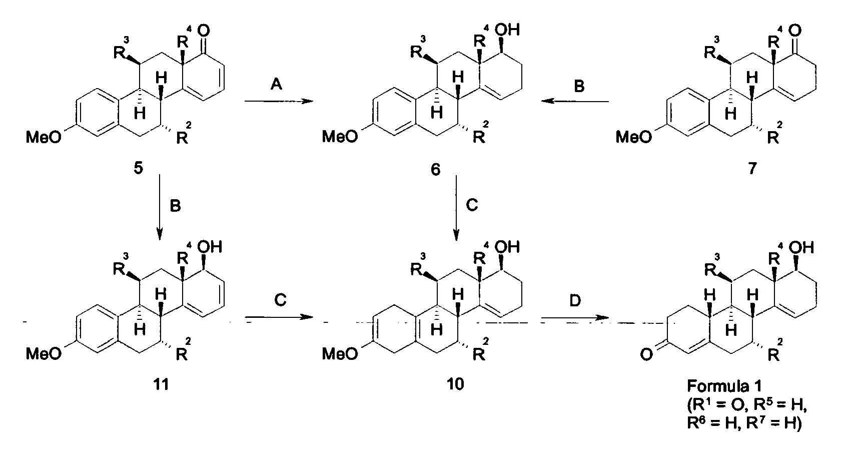

### Scheme 4 Experimental conditions (reagents and solvents mentioned are by way of example only): A: Lithium hexamethyl disilazide, methyl iodide or ethyl iodide. B: Lithium aluminum hydride. C: 1) Lithium/ammonia, 2) Hydrochloric acid, acetone.

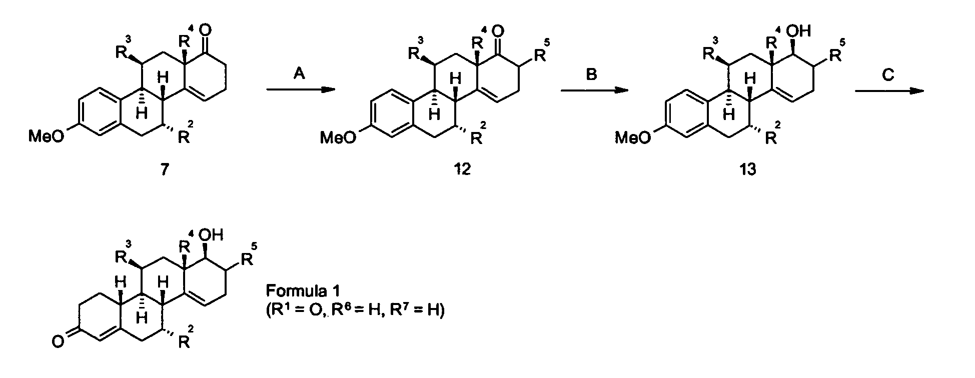

A 3-methoxy-D-homogona-1,3,5(10),14-tetraen-17a-one 7 can also be alkylated at the 17-position to introduce a 17-methyl or 17-ethyl group **(Scheme 4)** [for alkylation reactions, see e.g. Carey, F.A., Sundberg, R.J., "Advanced Organic Chemistry", Part B: Reactions and Synthesis, Chapter 1, Plenum Press, NY, 1990]. The alkylation reaction will produce a single diastereomer or a mixture of 17α- and 17β-isomers, in which case they have to be separated by chromatography or crystallisation. Optionally, the stereochemistry at C-17 can be inverted by deprotonation followed by hydrolysis. The resulting 17-alkylated 3-methoxy-D-homogona-1,3,5(10),14-tetraen-17a-one **12** can be reduced to alcohol **13.** Birch reduction of **13** and hydrolysis of the resulting Δ^{2,5(10),14} triene then provides a Δ¹⁴-D-Homo compound of Formula 1 (R¹ = oxo, R⁶ = H, R⁷ = H).

A 3-methoxy-D-homogona-1,3,5(10),14-tetraen-17a-one 7 can also be treated with an organometallic compound of formula R⁶M (R⁶ = methyl or ethyl, M = Li, MgCl, MgBr, etc.) to produce addition product **14** (Scheme 5, R⁶ = methyl or ethyl). The latter may be formed as a single diastereomer or together with the corresponding 17aα-OH,17aβ-alkyl isomer in which case they have to be separated by chromatography or crystallisation. A 17aα-methyl compound **14** (R⁶ = Me) can also be obtained by reaction of 7 with e.g. trimethylsulfonium iodide and base to give the 17a-epoxide **15** followed by ring opening by reaction with a reducing agent.
Birch reduction of **14** and hydrolysis of the resulting Δ^{2,5(10),14} trienes then provides a Δ¹⁴ -D-Homo compound of Formula 1 (R¹ = oxo, R⁵ = H, R⁶ = Me or Et, R⁷ = H).

### Scheme 5 Experimental conditions (reagents and solvents mentioned are by way of example only): A: Alkyl magnesium bromide. B: 1) Lithium/ammonia, 2) Hydrochloric acid, acetone. C: Trimethylsulfonium iodide, potassium tert-butoxide. D: Lithium aluminum hydride.

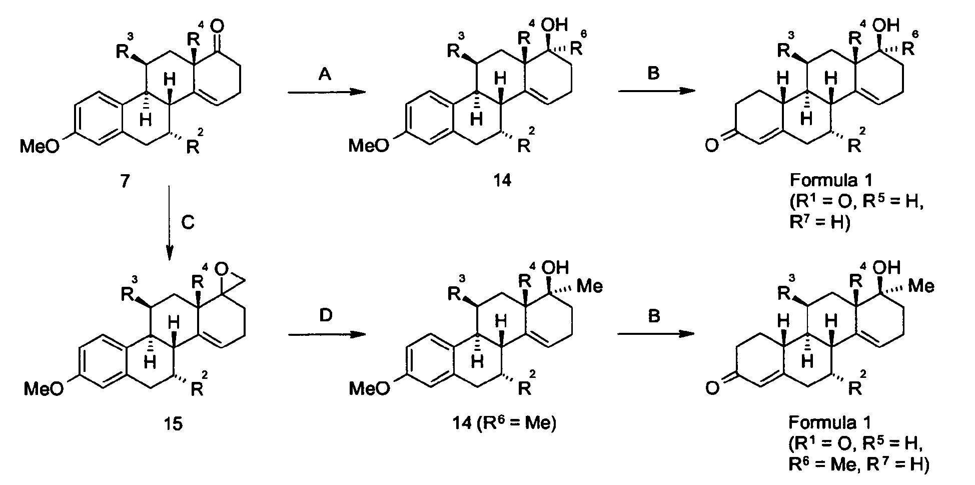

Compounds of the invention in which R⁶ is (C₂₋₃) alkenyl, or (C₂₋₃) alkynyl are obtained as follows. A (17aβ)-17a-hydroxy-D-homoestra-4,14-dien-3-one derivative of Formula 1 (R¹ = oxo, R⁵ = H, R⁶ = H, R⁷ = H) is oxidised to diketone **16 (Scheme 6).** Selective protection of the 3-oxo group, *e.g*. as cyclic 3-(1,2-ethanediyl dithioacetal), results in synthetic intermediate **17.** Reaction of the latter with R⁶M [R⁶ is (C₂₋₃) alkenyl, or (C₂₋₃) alkynyl, M = Li, Na, K, MgX etc.] gives an addition product **18** which may be formed as a single diastereomer or together with the corresponding 17aα-OH,17aβ-alkenyl or alkynyl isomer in which case they have to be separated by chromatography or crystallisation. Deprotection of compounds **18** provides a Δ¹⁴-D-Homo compound of Formula 1 [R¹ = oxo, R⁵ = H, R⁶ = (C₂₋₃) alkenyl, or (C₂₋₃) alkynyl, R⁷= H). The method can also be used for compounds with R⁶ is methyl or ethyl.

### Scheme 6 Experimental conditions (reagents and solvents mentioned are by way of example only): A: N-methylmorpholine oxide, tetrapropylammonium perruthenate, acetone. B: Ethanedithiol, boron trifluoride diethyl etherate. C: E.g. acetylene, potassium tert-butoxide. D: Periodic acid.

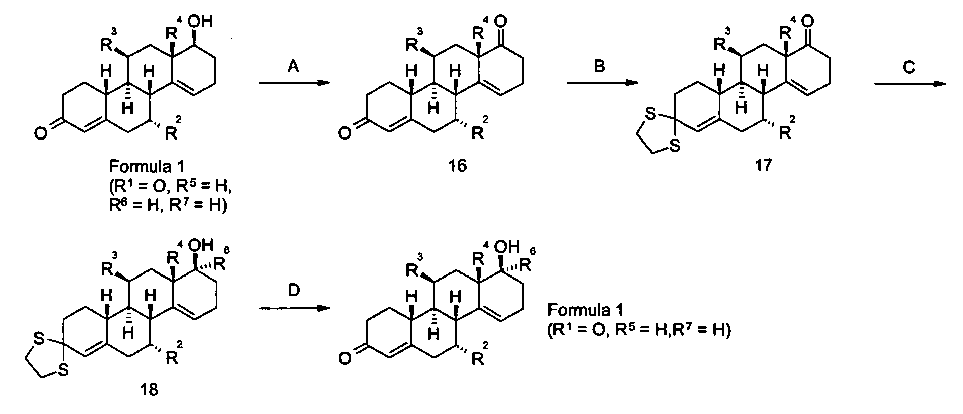

Compounds of the invention in which R¹ is (H,H), (H,OH), NOR, with R being hydrogen, (C₁₋₆) alkyl, (C₁₋₆) acyl, are obtained, by using methods known in the art, from compounds of Formula I in which R¹ is oxo.
Compounds of the invention in which R⁷ is (C₁₋₁₅) acyl are obtained, by using methods known in the art, from compounds of formula I in which R⁷ is hydrogen.

The invention will be further explained hereinafter with reference to the following Examples.

### EXAMPLES

### Example 1

### (7α,17αβ)-17a-Hydroxy-7-methyl-D-homoestra-4,14-dien-3-one.

**i)** -A solution of (7α)-3-methoxy-7-methylestra-1,3,5(10),14-tetraen-17-one [Segaloff, A. et al, Steroids 22, 99 (1973); 10.46 g] and trimethylsilyl cyanide (14.1 mL) in dry dichloromethane (106 ml) was treated with dry zinc iodide (0.035 g). After 1.5 h stirring, the reaction mixture was poured into a saturated aqueous solution of sodium hydrogencarbonate and the product was extracted into ethyl acetate. The combined organic phases were washed with brine, dried over sodium sulfate and concentrated under reduced pressure, to give (7αβ)-3-methoxy-7-methyl-17-[(trimethylsilyl)oxy]estra-1,3,5(10),14-tetraen-17-carbonitrile (13.5 g). The product was used in the following step without further purification.
**ii)** - A solution of the product obtained in the previous step (5.00 g) in dry tetrahydrofuran (28 mL) was added dropwise to a suspension of lithium aluminum hydride (0.91 g) in tetrahydrofuran (56 mL). The reaction mixture was stirrred at room temperature for 27 h; additional portions of lithium aluminum hydride (0.85 g and 0.84 g) were added after 3 h and 23 h, respectively. After cooling, the reaction mixture was quenched by addition of a saturated aqueous solution of sodium sulfate. Ethyl acetate was added, and the mixture was filtered over decalite (diatomaceous earth). The filtrate was concentrated under reduced pressure, to give (7α)-17-(aminomethyl)-3-methoxy-7-methyl-17-[(trimethylsilyl)oxy]estra-1,3,5(10),14-tetraene (4.00 g). The product was used in the following step without further purification.
**iii)** - Water (15 ml) was added to a solution of the product obtained in the previous step (4.00 g) in acetic acid (91 mL). The mixture was cooled to 0 °C, and treated with a solution of sodium nitrite (3.64 g) in water (15 ml). Additional acetic acid (35 ml) was added and the reaction mixture was stirred at room temperature overnight. The mixture was concentrated and the residue was poured into a saturated aqueous solution of sodium hydrogencarbonate. The product was extracted into ethyl acetate; the combined organic phases were washed with brine, dried over sodium sulfate and concentrated under reduced pressure. Column chromatography (heptane/ethyl acetate 85:5) afforded (7α)-3-methoxy-7-methyl-D-homoestra-1,3,5(10),14-tetraen-17a-one (0.71 g).
**iv)** - A solution of the product obtained in the previous step (0.71 g) in dry tetrahydrofuran (6 mL) was added dropwise to an ice-cooled suspension of lithium aluminum hydride (0.25 g) in tetrahydrofuran (3 mL). After 2 h stirring, the reaction mixture was quenched by addition of a saturated aqueous solution of sodium sulfate. Ethyl acetate was added, and the mixture was filtered over dicalite. The filtrate was concentrated under reduced pressure to produce (7α,17aβ)-3-methoxy-7-methyl-D-homoestra-1,3,5(10),14-tetraen-17a-ol (0.74 g). The product was used in the following step without further purification.
**v)** - A solution of the product obtained in the previous step (0.74 g) in dry tetrahydrofuran (10 mL) was added to a refluxing solution of lithium (0.47 g) in liquid ammonia (15 mL). After 1 h stirring, *tert*-butanol (1.44 ml) was added and stirring was continued for 30 min. Dry ethanol was added and the ammonia was allowed to evaporate. Water was added and the product was extracted into ethyl acetate. The combined organic phases were washed with a saturated aqueous solution of ammonium chloride and brine, dried over sodium sulfate and concentrated under reduced pressure, to give (7α,17αβ)-3-methoxy-7-methyl-D-homoestra-2,5(10),14-trien-17a-ol (0.79 g). The product was used in the following step without further purification.
**vi)** - A solution of the product obtained in the previous step (0.69 g) in acetone (9 mL) was treated with hydrochloric acid (6 M, 0.71 mL). After 2 h stirring at room temperature, the reaction mixture was neutralized with a saturated aqueous solution of sodium hydrogencarbonate. The product was extracted into ethyl acetate; the combined organic phases were washed with brine, dried over sodium sulfate and concentrated under reduced pressure. Column chromatography (toluene/ethyl acetate 8:2) and crystallization afforded (7α,17aβ)-17a-hydroxy-7-methyl-D-homoestra-4,14-dien-3-one (0.17 g), ¹H-NMR (CDCl₃) δ 5.85 (m, 1H), 5.26 (m, 1H), 3.51 (m, 1H), 2.47 (m, 2H), 1.72 (m, 2H), 1.09 (s, 3H), 0.81 (d, 3H, J = 7.1 Hz).

### Example 2

### (7α,17aβ)-7-Ethyl)-17a-hydroxy-D-homoestra-4,14-dien-3-one.

**i)** - A solution of diisopropylamine (16.3 mL) in dry tetrahydrofuran (113 mL) was cooled to -30 °C. n-BuLi (1.6 M solution in hexanes, 66.5 mL) was added dropwise (T < -10 °C) and stirring was continued at -30 °C for 10 min. The reaction mixture was cooled to -75 °C and a solution of (7α)-7-ethyl-3-methoxyestra-1,3,5(10),14-tetraen-17-one [WO 2001005806; 10.0 g] in dry tetrahydrofuran (153 mL) was added dropwise. Stirring was continued for 1 h. Chlorotrimethylsilane (12.1 mL) was added and the temperature was allowed to raise to room temperature in 20 min. After cooling to 0 °C, the reaction mixture was quenched with a saturated aqueous solution of ammonium chloride. The product was extracted into ethyl acetate; the combined organic phases were washed with brine, dried over sodium sulfate and concentrated under reduced pressure, to give (7α)-7-ethyl-3-methoxy-17-[(trimethylsilyl)oxy]estra-1,3,5(10),14,16-pentaene (12.05 g). The product was used in the following step without further purification.
**ii)** - A solution of the product obtained in the previous step (12.05 g) and diiodomethane (10.2 ml) in dry dichloromethane (188 mL) was cooled to 0 °C. A solution of diethylzinc in hexane (15 % wt., 114 mL) was added (T < 5 °C) and the reaction mixture was stirred for 1 h at room temperature. Ice was added and the reaction mixture was poured into a saturated aqueous solution of ammonium chloride. The product was extracted into ethyl acetate; the combined organic phases were washed with brine, dried over sodium sulfate, and concentrated under reduced pressure, to give (7α)-7-ethyl-3-methoxy-16,17-methylene-17-[(trimethylsilyl)oxy]estra-1,3,5(10),14-tetraene (12.96 g). The product was used in the following step without further purification.
**iii)** - A solution of the product obtained in the previous step (10.0 g) in dry dimethylformamide (51 mL) was added to a solution of iron(III) chloride (12.3 g) in the same solvent (51 mL), cooled to 0 °C. The reaction mixture was stirred for 1.5 h at room temperature and then, under cooling, quenched with aqueous hydrochloric acid (2 M). Water was added and the product was extracted into ethyl acetate. The combined organic phases were washed with brine, dried over sodium sulfate and concentrated under reduced pressure. Column chromatography (heptane/ethyl acetate 8:2) afforded (7α)-7-ethyl-3-methoxy-D-homoestra-1,3,5(10),14,16-pentaen-17a-one (5.10 g).
**iv)** - A solution of the product obtained in the previous step (3.90 g) in dry tetrahydrofuran (21.5 mL) was added dropwise to an ice-cooled suspension of lithium aluminum hydride (1.09 g) in tetrahydrofuran (21.5 mL) (T < 10 °C). After stirring of the mixture at 0 °C for 2 h, the reaction was quenched by addition of a saturated aqueous solution of sodium sulfate. Ethyl acetate was added, and the mixture was filtered over dicalite. The filtrate was concentrated under reduced pressure. Column chromatography (toluene/ethyl acetate 95:5) afforded (7α,17aβ-7-ethyl-3-methoxy-D-homoestra-1,3,5(10),14,16-pentaen-17a-ol (1.45 g).
**v)** - A solution of the product obtained in the previous step (1.45 g) in dry tetrahydrofuran (25 mL) was added to a refluxing solution of lithium (1.81 g) in liquid ammonia (101 mL). After 1 h stirring, *tert*-butanol (2.3 mL) was added and stirring was continued for 30 min. Dry ethanol was added and the ammonia was allowed to evaporate. Water was added and the product was extracted into ethyl acetate. The combined organic phases were washed with brine, dried over sodium sulfate and concentrated under reduced pressure, to give (7α,17aβ)-7-ethyl-3-methoxy-D-homoestra-2,5(10),14-trien-17a-ol (1.33 g). The product was used in the following step without further purification.
**vi)** - A solution of the product obtained in the previous step (1.61 g) in acetone (118 mL) was treated with hydrochloric acid (4 M, 6.1 mL). After 1 h stirring at room temperature, the reaction mixture was poured into a saturated aqueous solution of sodium hydrogencarbonate. The product was extracted into ethyl acetate; the combined organic phases were washed with brine, dried over sodium sulfate and concentrated under reduced pressure. Column chromatography (toluene/ethyl acetate 8:2) afforded (7α,17aβ)-7-ethyl-17a-hydroxy-D-homoestra-4,14-dien-3-one (0.68 g),¹H-NMR (CDCl₃) δ 5.86 (m, 1 H), 5.15 (m, 1 H), 3.50 (m, 1 H), 1.09 (s, 3H), 0.90 (m, 3H).

### Example 3

### (7α,17α,17aβ)-17a-Hydroxy-7,17-dimethyl-D-homoestra-4,14-dien-3-one.

**i)** - Lithium hexamethyl disilazide (1.0 M solution in tetrahydrofuran, 6.8 mL) was dissolved in tetrahydrofuran (50 mL) and the solution was cooled to -40 °C. A solution of (7α)-3-methoxy-7-methyl-D-homoestra-1,3,5(10),14-tetraen-17a-one (Example 1, step iii; 2.1 g) in tetrahydrofuran (5 mL) was added and the mixture was stirred for 1 h at -40/-30 °C. Methyl iodide (1.9 g) was added and stirring was continued for 1 h at -30/-20 °C. The reaction mixture was quenched with a saturated aqueous solution of ammonium chloride. The product was extracted into ethyl acetate; the combined organic layers were washed with brine, dried over sodium sulfate and concentrated. Column chromatography (silica, toluene/ethyl acetate 9:1) and crystallisation (heptane) afforded (7α,17α)-3-methoxy-7,17-dimethyl-D-homoestra-1,3,5(10),14-tetraen-17a-one (0.9 g).
**ii)** - A solution of the product obtained in the previous step (0.9 g) in tetrahydrofuran (5 mL) was added to a suspension of lithium aluminum hydride (0.21 g) in tetrahydrofuran (25 mL), cooled to 0 °C. The reaction mixture was stirred for 1 h and quenched with a saturated aqueous solution of sodium sulfate. The mixture was stirred for 1.5 h and then filtered over Celite. The residue was washed with ethyl acetate and the filtrate was concentrated. Column chromatography (silica, heptane/ethyl acetate 6:1) afforded (7α,17α,17β)-3-methoxy-7,17-dimethyl-D-homoestra-1,3,5(10),14-tetraen-17a-ol (0.230 g).
**iii)** - Lithium (0.140 g, 20 mmol) was added to liquid ammonia (50 ml), cooled to 30°C, and the mixture was stirred for 30 min. A solution of the product obtained in the previous step (0.230 g) in tetrahydrofuran (5 ml) was added. The mixture was stirred for 1 h. *tert*-Butanol (3 mL) was slowly added. The mixture was stirred for 30 min and ethanol (10 mL) was slowly added. A saturated aqueous solution of sodium hydrogencarbonate was added, water was added and the mixture was extracted with ethyl acetate. The combined organic layers were washed with brine, dried over sodium sulfate, and concentrated to produce (7α,17α,17aβ)-3-methoxy-7,17-dimethyl-D-homoestm-2,5(10),14-trien-17a-ol. The product was used in the following step without further purification.
**iv)** - A solution of the crude material obtained in the previous step in acetone (100 mL) was treated with aqueous hydrochoric acid (4 M, 5 mL). The mixture was stirred for 1 h and quenched with sodium hydrogencarbonate. The acetone was evaporated and the aqueous layer was extracted with ethyl acetate. The combined organic layers were washed with brine, dried over sodium sulfate, and concentrated. Crystallisation (heptane) gave (7α,17α,17aβ)-17a-hydroxy-7,17-dimethyl-D-homoestra-4,14-dien-3-one (0.090 g), ¹H-NMR (CDCl₃) δ 5.85 (bs, 1H), 5.21 (m, 1H), 3.10 (d, 1 H, J = 9.4 Hz), 2.53-2.40 (m), 2.40-2.14 (m), 2.13-1.95 (m), 1.90-1.76 (m), 1.63-1.10 (m), 1.09 (s, 3H), 1.04 (d, 3H, J = 6.0 Hz), 0.82 (d, 3H, J = 6.4 Hz).

### Example 4

### (7α,17aβ)-17a-Hydroxy-7,17a-dimethyl-D-homoestra-4,14-dien-3-one.

**i)** - A solution of diisopropylamine (233 mL) in dry tetrahydrofuran (650 mL) was cooled to -30 °C. n-BuLi (1.6 M solution in hexanes, 508 mL) was added dropwise while keeping the temperature below -10 °C. Stirring was continued at a temperature between -10 °C and - 20 °C for 15 min. The reaction mixture was cooled to -78 °C and a solution of (7α)-3-methoxy-7-methylestra-1,3,5(10),14-tetraen-17-one [Segaloff, A. et al, Steroids 22, 99 (1973); 48.6 g] in dry tetrahydrofuran (650 mL) was added dropwise. Stirring was continued for 1 h. The cooling bath was replaced by a water bath (T = 20 °C) and chlorotrimethylsilane (217 mL) was added dropwise. Stirring was continued for 15 min. The reaction mixture was cooled to 0 °C and quenched with a saturated aqueous solution of ammonium chloride. The product was extracted into ethyl acetate; the combined organic phases were washed with brine, dried over sodium sulfate and concentrated under reduced pressure, to give (7α)-3-methoxy-7-methyl-17-[(trimethylsilyl)oxy]estra-1,3,5(10),14,16-pentaene (87.1 g). The product was used in the following step without further purification.
**ii)** - A solution of the product obtained in the previous step (87.1 g) and diiodomethane (50.3 mL) in dry dichloromethane (900 mL) was cooled to 0 °C. A solution of diethylzinc in hexane (15 % wt., 665 ml) was added and the reaction mixture was stirred at 0 °C for 30 min. and at room temperature for 1.5 h. The reaction mixture was quenched with a saturated aqueous solution of ammonium chloride. After phase separation, the organic phase was washed with a saturated aqueous solution of ammonium chloride, water and brine, dried over sodium sulfate, and concentrated under reduced pressure, to give (7α)-3-methoxy-7-methyl-16,17-methylene-17-[(trimethylsilyl)oxy]estra-1,3,5(10),14-tetraene (93 g). The product was used in the following step without further purification.
**iii)** - Iron(III) chloride (53.5 g) was added to dry dimethylformamide (260 mL), cooled to 0 °C. A solution of the product obtained in the previous step (46 g) in dry dimethylformamide (260 mL) was added and the reaction mixture was stirred at 0 °C for 15 min. and then at room temperature for 17 h. The reaction mixture was poured into a mixture of aqueous hydrochloric acid and ice and the product was extracted into ethyl acetate. The combined organic phases were washed with water and brine, dried over sodium sulfate and concentrated under reduced pressure. Column chromatography (toluene/ethyl acetate 9:1) afforded (7α)-3-methoxy-7-methyl-D-homoestra-1,3,5(10),14,16-pentaen-17a-one (9.6 g).
**iv)** - A solution of the product obtained in the previous step (9.6 g) and triethylsilane (31.6 ml) in dry dichloromethane (100 mL) was treated with boron trifluoride diethyl etherate (31.6 mL). The reaction mixture was stirred at room temperature for 17h and then poured into a saturated aqueous solution of sodium hydrogencarbonate. The product was extracted into ethyl acetate; the combined organic phases were washed with water and brine, dried over sodium sulfate and concentrated under reduced pressure. Column chromatography (toluene/ethyl acetate 95:5) afforded (7α)-3-methoxy-7-methyl-D-homoestra-1,3,5(10),14-tetmen-17a-ol (7.9 g).
**v)** - A solution of the product obtained in the previous step (7.9 g) in acetone (225 mL), cooled to -5°C, was treated dropwise with Jones reagent (15.9 mL). After 20 min. stirring, the reaction mixture was quenched by addition of 2-propanol (38 mL). Stirring was continued for 30 min. whereafter the mixture was filtered over silica/dicalite. The residue was washed with ethyl acetate. The filtrate was dried over sodium sulfate and concentrated under reduced pressure. Column chromatography (toluene/ethyl acetate 99:1) afforded (7α)-3-methoxy-7-methyl-D-homoestra-1,3,5(10),14-tetraen-17a-one.
**vi)** - Potassium *tert*-butoxide (8.0 g) was added in portions to a solution of the product obtained in the previous step (5.0 g) in a mixture of dry dimethyl sulfoxide (90 mL) and dry tetrahydrofuran (30 mL), containing trimethylsulfonium iodide (11.0 g). The reaction mixture was stirred at room temperature for 2 h and then quenched with an aqueous solution of ammonium chloride. The product was extracted into ethyl acetate; the combined organic phases were washed with an aqueous solution of ammonium chloride, water and brine, dried over sodium sulfate, and concentrated under reduced pressure, to obtain (7α,17aβ)-3-methoxy-7-methylspiro-D-homoestra-1,3,5(10),14-tetraene[17a,2']oxirane (5.2 g). The product was used in the following step without further purification.
**vii)** - A solution of the product obtained in the previous step (5.2 g) in dry tetrahydrofuran (150 mL) was added dropwise to an ice-cooled suspension of lithium aluminum hydride (1.5 g) in tetrahydrofuran (200 mL). After 1 h stirring at room temperature, the reaction mixture was quenched with a saturated aqueous solution of sodium sulfate. The mixture was filtered over dicalite. The filtrate was dried over sodium sulfate and concentrated under reduced pressure. Column chromatography (toluene/ethyl acetate 9:1) afforded (7α,17aβ)-3-methoxy-7,17a-dimethyl-D-homoestra-1,3,5(10),14-tetraen-17a-ol (2.4 g).
**viii)** - The product obtained in the previous step (2.4 g) in dry tetrahydrofuran (40 mL) and *tert*-butanol (1.6 mL) were added to refluxing liquid ammonia (120 mL). Lithium granulate was added in portions to the solution until the blue colour persisted for at least 30 min. Then, the reaction mixture was quenched with ethanol and the ammonia was allowed to evaporate. Water was added and the product was extracted into ethyl acetate. The combined organic phases were washed with water and brine, dried over sodium sulfate and concentrated under reduced pressure, to give (7α,17aβ)-3-methoxy-7,17a-dimethyl-D-homoestra-2,5(10),14-trien-17a-ol (2.7 g). The product was used in the following step without further purification.
**ix)** - A solution of the product obtained in the previous step (2.7 g) in acetone (54 mL) was treated with hydrochloric acid (2 M, 2.7 mL). After 1 h stirring at room temperature, the reaction mixture was quenched with a saturated aqueous solution of sodium hydrogencarbonate. The product was extracted into ethyl acetate; the combined organic phases were washed subsequently with water, a saturated aqueous solution of sodium hydrogencarbonate, water, and brine, dried over sodium sulfate and concentrated under reduced pressure. Column chromatography (toluene/ethyl acetate 9:1) afforded (7α,17aβ)-17a-hydroxy-7,17a-dimethyl-D-homoestra-4,14-dien-3-one (1.22 g), m.p. 166 °C, optical rotation (ethanol 100%, c=1) -20.0°, ¹H-NMR (CDCl₃) δ 5.92 (bs, 1H), 5.32 (bs, 1H), 2.63-1.91 (m), 1.82-1.11 (m), 1.28 (s, 3H), 0.87 (d, 3H).

### Example 5

### (3β,7α,17aβ)-7,17a-Dimethyl-D-homoestra-4,14-dien-3,17a-diol.

**i)** - A solution of (7α,17aβ)-17a-hydroxy-7,17a-dimethyl-D-homoestra-4,14-dien-3-one (Example 4; 0.628 g) in dry tetrahydrofuran (6 mL) was added to an ice-cooled suspension of lithium aluminum hydride (0.152 g) in tetrahydrofuran (30 mL). The cooling bath was removed and the reaction mixture was stirred for 1 h. The reaction mixture was quenched with a saturated aqueous solution of sodium sulfate (1.8 mL) and the mixture was stirred for 10 min. Diethyl ether (80 mL) and solid sodium sulfate (18 g) were added and stirring was continued for 10 min. Methanol (4.8 mL) was added and stirring was continued for another 30 min. The mixture was filtered over dicalite and the residue was washed with tetrahydrofuran. The filtrate was concentrated. The residue was purified by crystallisation (ether), column chromatography (silica, heptane/ethyl acetate 1/1), and again crystallisation (ether → heptane) to afford (3β,7α,17aβ)-7,17a-Dimethyl-D-homoestra-4,14-dien-3,17a-diol (0.290 g),¹H-NMR (CDCl₃) δ 5.38 (bs, 1 H), 5.23 (m, 1 H), 4.22 (bs, 1 H), 2.32- 2.02 (m), 1.98-1.86 (m), 1.76 (m, 1 H), 1.62 (m), 1.19 (s, 3H), 1.48-1.06 (m), 0.95 (m), 0.78 (d, 3H, J = 7.4 Hz).

### Example 6

### (7α,17aα)-17a-Hydroxy-7-methyl-D-homo-19-norpregna-4,14-dien-3-one.

**i)** - A solution of (7α)-3-methoxy-7-methyl-D-homoestra-1,3,5(10),14-tetraen-17a-one (Example 1, step iii; 1.5 g, 4.8 mmol) in tetrahydrofuran (50 mL) was cooled to -20 °C. Ethylmagnesium bromide (3.0 M solution, 16 mL, 48 mmol) was added slowly while keeping the temperature below -20 °C. The mixture was stirred at 0 °C for 1 h and then quenched with a saturated aqueous solution of ammonium chloride. The product was extracted into ethyl acetate. The combined organic layers were washed with brine, dried over sodium sulfate, and concentrated. The product was purified over silica (heptane/ethyl acetate 9:1) yielding (7α,17aα)-3-methoxy-7-methyl-D-homo-19-norpregna-1,3,5(10),14-tetraen-17a-ol (0.180 g).
**ii)** - Lithium (0.111 g, 15.9 mmol) was added to liquid ammonia (50 mL), cooled at -30 °C. The mixture was stirred for 30 min and a solution of the product obtained in the previous step (0.180 g, 0.53 mmol) in tetrahydrofuran (5 mL) and added. The mixture was stirred for 1 h. *tert*-Butanol (3 ml) was slowly added. Stirring was continued for 30 min and ethanol (10 ml) was slowly added. A saturated aqueous solution of sodium hydrogencarbonate was added, water was added and the mixture was extracted with ethyl acetate. The combined organic layers were washed with brine, dried over sodium sulfate, and concentrated, to produce (7α,17aα)-3-methoxy-7-methyl-D-homo-19-norpregna-2,5(10),14-trien-17a-ol. The product was used in the following step without further purification.
**iii)** - A solution of the product obtained in the previous step in acetone (100 mL) was treated with aqueous hydrochloric acid (4 M, 5 ml). The mixture was stirred for 1 h and quenched with sodium hydrogencarbonate. The acetone was evaporated and the aqueous layer was extracted with ethyl acetate. The combined organic layers were washed with brine and dried over sodium sulfate. The product was purified over silica (heptane/ethyl acetate 1:1) yielding (7α, 17aα)-17a-hydroxy-7-methyl-D-homo-19-norpregna-4,14-dien-3-one (0.125 mg), ¹H-NMR (CDCl₃) δ 5.86 (bs, 1H), 5.29 (bs, 1H), 2.53-2.23 (m), 2.14-1.94 (m), 1.79 (m, 1H), 1.69-1.46 (m), 1.36 (m), 1.23 (s, 3H), 1.17 (m), 0.97 (t, 3H, J = 7.4 Hz), 0.79 (d, 3H, J = 6.6 Hz).

### Example 7

### (17aβ)-17a-Hydroxy-D-homoestra-4,14-dien-3-one.

**i)** - A solution of diisopropylamine (125 mL) in tetrahydrofuran (1.0 L) was cooled to -25 °C. *n*-Butyllithium (2.5 M solution in hexane, 320 mL, 0.8 mol) was added at such a rate that the temperature was maintained below -20 °C. The mixture was stirred for 30 minutes at -30 °C and then cooled to -78 °C. A solution of 3-methoxyestra-1,3,5(10),14-tetraen-17-one [WO 2002048169; 68.6 g, 0.23 mol] in tetrahydrofuran (1.0 L) was added at such a rate that the temperature was maintained below -70°C and the resulting mixture was stirred for 1 h at -70 °C. Chlorotrimethylsilane (92 ml) was added dropwise. The mixture was allowed to warm to room temperature overnight and then poured in a saturated aqueous solution of ammonium chloride. The mixture was extracted with ethyl acetate. The combined organic phases were washed with brine, dried over sodium sulfate, and concentrated to afford 3-methoxy-17-[(trimethylsilyl)oxy]estra-1,3,5(10),14,16-pentaene (116.5 g, purity ca 40%).
**ii)** - Diiodomethane (54 mL) was added to a solution of the product of the previous step (58 g) in dichloromethane (1 L), and the mixture was cooled to 10°C. Diethylzinc (15 % weight solution in hexane, 600 mL) was added dropwise and the mixture was stirred overnight at room temperature. The reaction mixture was quenched by dropwise addition of a saturated aqueous solution of ammonium chloride. The resulting mixture was poured into a saturated aqueous solution of ammonium chloride and the product was extracted into ethyl acetate. The combined organic phases were washed with brine, dried over sodium sulfate, and concentrated to afford 3-methoxy-16,17-methylene-17-[(trimethylsilyl)oxy]estra-1,3,5(10),14-tetraene (65.9 g). The product was used in the following step without further purification.
**iii)** - Iron(III) chloride (150 g) was dissolved in dimethylformamide (600 mL) with ice/salt cooling (5-20 °C). A solution of the product obtained in the previous step (127.7 g) in dimethyl formamide (600 mL) was added at such a rate that the temperature was maintained below 10°C and the reaction mixture was stirred overnight at room temperature. The reaction mixture was poured into aqueous hydrochloric acid (2.5 M, 5 L) and the product was extracted into ethyl acetate. The combined organic phases were washed with water and brine, dried over sodium sulfate, and concentrated. Column chromatography (silica, heptane/ethyl acetate 9:1) gave 3-methoxy-D-homoestra-1,3,5(10),14,16-pentaen-17a-one (11.40 g).
**iv)** - A solution of the product obtained in the previous step (11.4 g) in dichloromethane (500 mL) was treated with triethylsilane (19.7 mL) and boron trifluoride diethyl etherate (48 %, 20 mL). The reaction mixture was stirred for 24 h [no conversion (¹H-NMR)]. Extra amounts of triethylsilane (19.7 mL) and boron trifluoride diethyl etherate (48 %, 20 mL) were added and the reaction mixture was stirred for another 70 h [no conversion (¹H-NMR)]. The reaction mixture was poured in a saturated aqueous solution of sodium hydrogencarbonate and extracted with ethyl acetate. The combined organic phases were washed with brine, dried on sodium sulfate, and concentrated affording starting material (11.5 g). It was dissolved in dichloromethane (500 mL), triethylsilane (19.7 mL) and boron trifluoride diethyl etherate (48 %, 20 mL) were added and the reaction mixture was stirred for 48 h [ca 50 % conversion (¹H-NMR)]. The reaction mixture was poured into a saturated aqueous solution of sodium hydrogencarbonate and extracted with ethyl acetate. The combined organic phases were washed with brine, dried over sodium sulfate, and concentrated. Column chromatography (silica, heptane/ethyl acetate 4:1) afforded (17aβ)-3-methoxy-D-homoestra-1,3,5(10),14-tetraen-17a-ol (2.9 g), and a mixture of starting material and product (6.9 g). Performing the reduction once more on the mixture afforded a second batch of product (2.1 g) which was combined with the first (5.0 g).
**v)** - A solution of the product obtained in the previous step (4.0 g) in tetrahydrofuran (100 mL) was added to liquid ammonia (ca. 125 mL), cooled to -78 °C. Lithium (1.9 g) was added and the solution was stirred for 30 min at -78 °C. A mixture of tetrahydrofuran (15 mL) and *tert*-butanol (15 g) was added within 15 min and the mixture was stirred for 2 h at -78 °C. Solid ammonium chloride (15 g) and ethyl acetate (100 mL) were added carefully. Water (200 mL) was added and the mixture was allowed to warm to room temperature. The product was extracted into ethyl acetate. The combined organic phases were washed with water and brine, dried over sodium sulfate, and concentrated affording (17aβ)-3-methoxy-D-homoestra-2,5(10),14-trien-17a-ol (3.8 g). The product was used in the following step without further purification.
**vi)** - A solution of the product obtained in the previous step (3.8 g) in acetone (150 mL) was treated with aqueous hydrochloric acid (1 M, 40 mL). The reaction mixture was stirred overnight at room temperature and subsequently quenched with sodium hydrogencarbonate. The acetone was evaporated and the mixture was extracted with ethyl acetate. The organic phase was washed with brine, dried on sodium sulfate and concentrated. Column chromatography (heptanes/ethyl acetate 3:1) afforded (17aβ)-17a-hydroxy-D-homoestra-4,14-dien-3-one (1.3 g),¹H-NMR (CDCl₃) δ5.90 (bs, 1 H), 5.35 (bs, 1H), 2.71-0.80 (m), 1.22 (s, 3H).

### Example 8

### (17aβ)-17a-Hydroxy-17a-methyl-D-homoestra-4,14-dien-3-one.

**i)** -A solution of (17aβ)-3-methoxy-D-homoestra-1,3,5(10),14-tetraen-17a-ol (Example 7, step iv; 5.0 g) in acetone (200 mL) was treated with *N*-methylmorpholine oxide (9.8 g) and tetrapropylammonium perruthenate (0.25 g). The mixture was stirred overnight at room temperature. The reaction mixture was filtered through Celite and concentrated affording 3-methoxy-D-homoestra-1,3,5(10),14-tetraen-17a-one (4.7 g). The product was used in the following step without further purification.
**ii)** - The product obtained in the previous step (4.7 g) was dissolved in a mixture of dimethyl sulfoxide (120 mL) and tetrahydrofuran (50 mL). Trimethylsulfonium iodide (9.24 g) and potassium *tert*-butoxide (6.76 g, 60.2 mmol) were added and the resulting dark brown solution was stirred for 70 h. The resulting mixture was diluted with a saturated aqueous solution of ammonium chloride and extracted with ethyl acetate. The combined organic phases were washed with water and brine, dried on sodium sulfate and concentrated affording a 1:1 mixture of (17aβ)-3-methoxyspiro-D-homoestra-1,3,5(10),14-tetraene[17a,2']oxirane and (17aα)-3-methoxyspiro-D-homoestra-1,3,5(10),14-tetraene[17a,2']oxirane (4.52 g). The product was used in the following step without further purification.
**iii)** - A solution of the product obtained in the previous step (4.52 g) in tetrahydrofuran (50 mL) was added dropwise to a suspension of lithium aluminum hydride (2.12 g) in tetrahydrofuran (150 mL), cooled to 0 °C. The reaction mixture was stirred for 2 h at room temperature. The mixture was carefully quenched with water (10 mL), allowed to stir overnight, and filtered through Celite. The filtercake was suspended and boiled in tetrahydrofuran (250 mL) and filtered, the filtrate was combined with the first filtrate. The combined filtrates were dried over sodium sulfate and concentrated. Column chromatography (silica, heptane/ethyl acetate 4:1) gave (17aβ)-3-methoxy-17a-methyl-D-homoestra-1,3,5(10),14-tetraen-17a-ol (1.8 g).
**iv)** - A solution of the product obtained in the previous step (1.6 g) in tetrahydrofuran (100 mL) was added to liquid ammonia (100 mL), cooled to -78 °C. Lithium granules (0.35 g) were added in portions and the reaction mixture was stirred for 1 h at -78 °C. The reaction was quenched by the addition of a mixture of 2-propanol (25 mL) and tetrahydrofuran (25 mL) and the reaction mixture was allowed to warm to room temperature overnight. Water was added and the product was extracted into ethyl acetate. The combined organic phases were washed with brine, dried over sodium sulfate and concentrated to afford (17aβ)-3-methoxy-17a-methyl-D-homoestra-2,5(10),14-trien-17a-ol (1.7 g). The product was used in the following step without further purification.
**v)** - A solution of the product obtained in the previous step (1.7 g) in a mixture of acetone (50 mL) and water (4 mL) was treated with aqueous hydrochloric acid (30 %, 3 mL) and the reaction mixture was stirred for 2 h at room temperature. The reaction mixture was neutralized using a saturated aqueous solution of sodium hydrogencarbonate and concentrated. Water was added and the product was extracted into ethyl acetate. The combined organic phases were washed with brine, dried over sodium sulfate and concentrated. Crystallisation (heptane/ethyl acetate 4: 1) afforded (17aβ)-17a-hydroxy-17a-methyl-D-homoestra-4,14-dien-3-one (0.800 g),¹H-NMR (CDCl₃) δ 5.84 (bs, 1H), 5.32 (bs, 1H), 2.59-1.91 (m), 1.79-1.31 (m), 0.92 (m, 1 H), 1.19 (s, 3H), 1.18 (s, 3H).

### Example 9

### (17aα)-17a-Hydroxy-D-homo-19-norpregna-4,14-dien-20-yn-3-one.

**i)** - N-methylmorpholine oxide (2.46 g) was added to a solution of (17aβ)-17a-hydroxy-D-homoestra-4,14-dien-3-one (Example 7, 2.0 g) in acetone (100 mL) and the mixture was stirred for 15 min. Tetrapropylammonium perruthenate (70 mg, 0.2 mmol) was added and the reaction mixture was stirred for 3 h. Then it was filtered over Celite, the residue was washed with ethyl acetate/acetone and the filtrate was concentrated. Column chromatography (silica, heptane/ethyl acetate 2:1) gave D-homoestra-4,14-diene-3,17a-dione (0.400 g).
**ii)** - Ethanedithiol (0.087 g) was added to a solution of the product obtained in the previous step (0.4 g, 1.4 mmol) in methanol (25 mL) and the mixture was cooled to 0 °C. Boron trifluoride diethyl etherate (99 mg, 0.7 mmol) was added and the reaction mixture was stirred overnight. The mixture was concentrated and the solid was purified by column chromatography (silica, heptane/ethyl acetate 9:1) to produce D-homoestra-4,14-diene-3,17a-dione cyclic 3-(1,2-ethanediyl dithioacetal) (0.3 g).
**iii)** - A solution of potassium *tert*-butoxide (0.19 g) in tetrahydrofuran (50 mL) was cooled to 10°C. Acetylene was passed through for 10 min. The mixture was cooled to 0 °C and a solution of the product obtained in the previous step (0.3 g) in tetrahydrofuran (10 mL) was added at 0 °C while acetylene was still being passed through. Acetylene addition was continued for another 15 min after which the reaction was stirred for another hour at room temperature. The mixture was quenched with a saturated aqueous solution of sodium hydrogencarbonate and the product was extracted into ethyl acetate. The combined organic layers were washed with brine, dried over sodium sulfate, and concentrated. Column chromatography (silica, heptane/ethyl acetate 9: 1) gave a mixture of (17aα)-D-homo-19-norpregna-4,14-dien-20-yn-3-one cyclic (1,2-ethanediyl dithioacetal) and (17aβ)-D-homo-19-norpregna-4,14-dien-20-yn-3-one cyclic (1,2-ethanediyl dithioacetal) (0.2 g).
**iv)** - A solution of the product obtained in the previous step (0.085 g) in methanol (10 mL) was cooled to 0 °C. A solution of periodic acid (0.027 g) in water (0.5 mL) was slowly added. The mixture was stirred for 1 h and quenched with a saturated aqueous solution of sodium sulfite. The product was extracted into ethyl acetate; the combined organic layers were washed with brine, dried over sodium sulfate and concentrated. Preparative HPLC (heptane/2-propanol) gave (17aα)-17a-hydroxy-D-homo-19-norpregna-4,14-dien-20-yn-3-one (0.030 g), ¹H-NMR (CDCl₃) δ 5.84 (m, 1 H), 5.30 (m, 1 H), 2.59-1.16 (m), 2.48 (s, 1 H), 1.23 (s, 3H), 1.08-0.85 (m).

### Example 10

### Androgenic and progestagenic agonistic activity of the compounds of the invention

The compounds according to the invention were tested for androgenic activity and for progestagenic activity.
The transactivative androgen agonistic activity of the compounds of the invention was measured in Chinese hamster ovary cells (CHO) transfected with the human androgen receptor (hAR), in combination with a mouse mammary tumor virus (MMTV), and luciferase receptor gene (incubation time 16 h, temperature 37°C) and compared with the activity of 5α-dihydrotestosterone [according to the procedure described by Schoonen, W.G.E.J. et al., Analyt. Biochem. 261, 222-224 (1998)].
The transactivative progestagenic agonistic activity of the compounds of the invention was measured in Chinese hamster ovary cells (CHO) transfected with the human progesterone receptor B (hPRB), in combination with a mouse mammary tumor virus (MMTV), and luciferase receptor gene (incubation time 16 h, temperature 37 °C) and compared with the activity of (16α)-16-ethyl-21-hydroxy-19-norpregn-4-ene-3,20-dione [according to the procedure described by Schoonen, W.G.E.J. et al., Analyt. Biochem. 261, 222-224 (1998)]. Results are collected in Table 1.

**Table 1. Androgenic and progestational data of a compound of the invention.**

| **Compound** | **Androgen activity** | **Progestagen activity** |
|---|---|---|
| | Relative agonistic activity (%) versus 5α-dihydrotestosterone | Relative agonistic activity (%) versus (16α)-16-ethyl-21-hydroxy-19-norpregn-4-ene-3,20-dione |
| Compound of Example 2 | 30.5 | 0.47 |
| Compound of Example 3 | 10.2 | 47.4 |
| Compound of Example 4 | 106.9 | 133.7 |
| Compound of Example 5 | 36.2 | 6.4 |
| Compound of Example 6 | 13.2 | 91.2 |
| Compound of Example 8 | 157.9 | 228.4 |
| Compound of Example 9 | 12.3 | 16.0 |

### Example 11

### Determination of liver safety: the BSP retention assay

The primary aim of this test was the evaluation of liver function, by measuring the clearance of a single, intravenously injected, dose of bromosulfophthalein (BSP) after 7 days (once daily) per oral treatment of male castrated rabbits with a compound of the invention.
Briefly, this test is performed in mature male castrated New Zealand White rabbits. At least two weeks after castration animals are treated for seven days (once daily) with tablets, or arachis oil containing 5% (v/v) ethanol, resulting in a dose of 10 mg/kg/day of a test compound. Twenty-four hours after the last administration the rabbits were light sedated with 0.15 ml Hypnorm intramuscular, BSP was injected (15 mg/kg intravenous, dissolved in 5% (w/v) mannitol/H₂O). Blood is collected at 5, 10, 15 and 20 minutes, BSP content in plasma is determined with a spectrophotometer. The t½ of BSP elimination from the blood is calculated from the obtained data.

**Table 2. BSP data of a compound of the invention.**

| **Compound** | **Vehicle** | **T½ BSP (minutes)** |
|---|---|---|
| Compound of Example 4 | Tablet | 5.5 |
| Testosterone | Tablet | 4.4 |
| 17α-Methyltestosterone | Tablet | 6.0 |
| Reference compound* | Tablet | 8.8 |
| Compound of Example 4 | Arachis oil + 5% ethanol | 4.9 |
| Testosterone | Arachis oil + 5% ethanol | 4.2 |
| 17α-Methyltestosterone | Arachis oil + 5% ethanol | 5.6 |
| Reference compound* | Arachis oil + 5% ethanol | 8.5 |

| | | |
|---|---|---|
| *(7α,17β)-17-Hydroxy-7,17-dimethylestra-4,14-dien-3-one. | | |

## Claims

1. A compound having formula 1 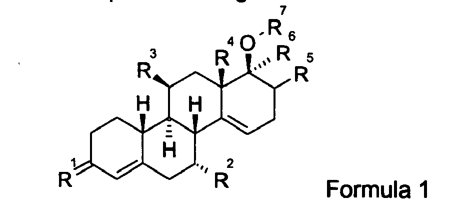 wherein,
R¹ is O, (H,H), (H,OH), or NOR, with R being hydrogen, (C₁₋₆) alkyl or (C₁₋₆) acyl;
R² is hydrogen, methyl, ethyl, or ethenyl;
R³ is hydrogen, methyl, ethyl, ethenyl, fluoro, or chloro;
R⁴ is methyl, or ethyl;
R⁵ is hydrogen, methyl, or ethyl;
R⁶ is hydrogen, methyl, ethyl, (C₂₋₃) alkenyl, or (C₂₋₃) alkynyl;
R⁷ is hydrogen or (C₁₋₁₅) acyl;
and at least one of R⁵ or R⁶ is hydrogen;
or a pharmaceutically acceptable salt thereof.

2. The compound according to claim 1, **characterized in that**
R¹ is O or (H,OH);
R² is hydrogen, methyl, ethyl;
R³ is hydrogen, methyl;
R⁴ is methyl;
R⁵ is hydrogen, methyl;
R⁶ is hydrogen, methyl, ethyl, or (C₂₋₃) alkynyl;
R⁷ is hydrogen or (C₁₋₁₅) acyl.

3. The compound according to claim 2, **characterized in that**
R² is hydrogen, methyl;
R³ is hydrogen;
R⁵ is hydrogen;
R⁶ is hydrogen, methyl, ethyl, or ethynyl.

4. The compound according to any one of claim 1-3, **characterized in that**
R¹ is O;

5. The compound according to claim 4, **characterized in that** it is (7α,17aβ)-17a-hydroxy-7-methyl-D-homoestra-4,14-dien-3-one.

6. The compound according to claim 4, **characterized in that** it is (7α,17aβ)-17a-hydroxy-7,17a-dimethyl-D-homoestra-4,14-dien-3-one.

7. The compound according to claim 4, **characterized in that** it is (17aβ)-17a-hydroxy-17a-methyl-D-homoestra-4,14-dien-3-one.

8. The compound according to any one of claim 1-7 for therapy

9. A pharmaceutical composition comprising a compound according to any one of claim 1-7

10. Use of a compound according to any one of claim 1-7 for the manufacture of a medicine for a treatment to increase androgen levels.

11. Use of a compound according to claim 10 for the manufacture of a medicine for androgen supplementation, for testosterone replacement or for benign prostate hypertrophy (BPH).

## Patentansprüche

1. Verbindung gemäss Formel 1 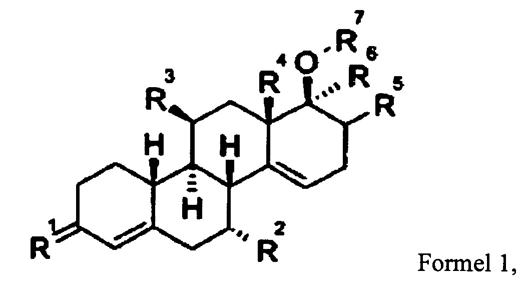 wobei,
R¹ O, (H,H), (H,OH), oder NOR ist, wobei R Wasserstoff, (C₁₋₆)Alkyl oder (C₁₋₆)Acyl ist;
R² Wasserstoff, Methyl, Ethyl, oder Ethenyl ist;
R³ Wasserstoff, Methyl, Ethyl, Ethenyl, Fluor oder Chlor ist;
R⁴ Methyl, oder Ethyl ist;
R⁵ Wasserstoff, Methyl, oder Ethyl ist;
R⁶ Wasserstoff, Methyl, Ethyl, (C₂₋₃)Alkenyl, oder (C₂₋₃)Alkynyl ist;
R⁷ Wasserstoff oder (C₁₋₁₅)Acyl ist;
und mindestens einer von R⁵ oder R⁶ Wasserstoff ist;
oder ein pharmazeutisch verträgliches Salz davon.

2. Verbindung gemäss Anspruch 1, **dadurch gekennzeichnet, dass**
R¹O oder (H,OH) ist;
R² Wasserstoff, Methyl, Ethyl ist;
R³ Wasserstoff, Methyl ist;
R⁴ Methyl ist;
R⁵ Wasserstoff, Methyl ist;
R⁶ Wasserstoff, Methyl, Ethyl, oder (C₂₋₃)Alkynyl ist;
R⁷ Wasserstoff oder (C₁₋₁₅)Acyl ist.

3. Verbindung gemäss Anspruch 2, **dadurch gekennzeichnet, dass**
R² Wasserstoff, Methyl ist;
R³ Wasserstoff ist;
R⁵ Wasserstoff ist;
R⁶ Wasserstoff, Methyl, Ethyl, oder Ethynyl ist.

4. Verbindung gemäss irgendeinem der Ansprüche 1-3, **dadurch gekennzeichnet, dass**
R¹O ist.

5. Verbindung gemäss Anspruch 4, **dadurch gekennzeichnet, dass** sie (7α,17aβ)-17a-Hydroxy-7- methyl-D-homoestra-4,14-dien-3-on ist.

6. Verbindung gemäss Anspruch 4, **dadurch gekennzeichnet, dass** sie (7α,17aβ)-17a-Hydroxy-7,17a-dimethyl-D-homoestra-4,14-dien-3-on ist.

7. Verbindung gemäss Anspruch 4, **dadurch gekennzeichnet, dass** sie (17aβ)-17a-Hydroxy-17a- methyl-D-homoestra-4,14-dien-3-on ist.

8. Verbindung gemäss irgendeinem der Ansprüche 1-7 für die Therapie.

9. Pharmazeutische Zusammensetzung umfassend eine Verbindung gemäss irgendeinem der Ansprüche 1-7.

10. Verwendung einer Verbindung gemäss irgendeinem der Ansprüche 1-7 für die Herstellung eines Medikamentes für eine Behandlung, um Androgen-Niveaus zu erhöhen.

11. Verwendung einer Verbindung gemäss Anspruch 10 für die Herstellung eines Medikamentes für die Androgen-Ergänzung, für den Testosteron-Ersatz oder für gutartige Prostata-Hypertrophie (BPH).

## Revendications

1. Un composé de formule : 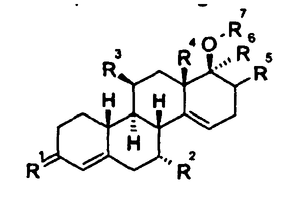 dans laquelle
- R¹ est O, (H,H), (H, OH), ou NOR où R est un atome d'hydrogène ou un radical (C₁₋₁₆)-alkyl ou (C₁₋₆)-acyl;
- R² est un atome d'hydrogène ou un radical méthyl, éthyl ou éthényl
- R³ est un atome d'hydrogène ou un radical méthyl, éthyl, éthényl, fluoro ou chloro;
- R⁴ est un radical méthyl ou éthyl
- R⁵ est un atome d'hydrogène ou un radical méthyl ou éthyl;
- R⁶ est un atome d'hydrogène ou un radical méthyl, (C₂₋₃) éthyl, (C₂₋₃) alkynyl;
- R⁷ un atome d'hydrogène, (C₁₋₁₅)-acyl;
et au moins un des R⁵ ou R⁶ est un atome d'hydrogène;
ou les sels pharmaceutiquement acceptables en dérivant.

2. Le composé selon la revendication 1, **caractérisé en ce que**:
- R¹ est O ou (H,OH);
- R² est un atome d'hydrogène ou un radical méthyl, éthyl;
- R³ est un atome d'hydrogène ou un radical méthyl;
- R⁴ est un radical méthyl;
- R⁵ est un atome d'hydrogène ou un radical méthyl;
- R⁶ est un atome d'hydrogène ou un radical méthyl, éthyl ou (C₂₋₃) alkynyl;
- R⁷ est un atome d'hydrogène ou un radical (C₁₋₁₅) acyl.

3. Le composé selon la revendication 2, **caractérisé en ce que**:
- R² est un atome d'hydrogène ou un radical méthyl;
- R³ est un atome d'hydrogène;
- R⁵ est un atome d'hydrogène;
- R⁶ est un atome d'hydrogène ou un radical méthyl, éthyl ou éthynyl;

4. Le composé selon l'une des quelconques revendications 1-3, **caractérisé en ce que** R¹ est O

5. Le composé selon la revendication 4, **caractérisé en ce qu'**il consiste en (7α,17aβ)-17a-hydroxy-7-méthyl-D-homoestra-4,14-dien-3-one.

6. Le composé selon la revendication 4, **caractérisé en ce qu'**il consiste en (7α,17aβ)-17a-hydroxy-7,17a-diméthyl-D-homoestra-4,14-dien-3-one.

7. Le composé selon la revendication 4, **caractérisé en ce qu'**il consiste en (17aβ)-17a-hydroxy-17a-méthyl-D-homoestra-4,14-dien-3-one.

8. Le composé selon l'une des quelconques revendications 1-7, pour traitements thérapeutiques

9. Une composition pharmaceutique comprenant un composé selon l'une quelconque des revendications 1-7.

10. Utilisation d'un composé selon l'une quelconque des revendications 1-7 pour la fabrication d'un médicament pour traitements en vue d'augmenter le taux d'androgène.

11. Utilisation d'un composé selon la revendication 10 pour la fabrication d'un médicament pour supplémenter les androgènes, remplacer les testostérones ou l'hypertrophie bénigne de la prostate (BPH).
